(19) Europäisches Patentamt / European Patent Office / Office européen des brevets

(11) **EP 3 462 177 B1**

(12) **EUROPEAN PATENT SPECIFICATION**

(45) Date of publication and mention of the grant of the patent:
**16.04.2025 Bulletin 2025/16**

(21) Application number: **18197205.0**

(22) Date of filing: **27.09.2018**

(51) International Patent Classification (IPC):
*G01N 35/10* (2006.01)    *G01N 35/00* (2006.01)

(52) Cooperative Patent Classification (CPC):
**G01N 35/0099; G01N 35/10;** G01N 2035/1032

(54) **DISPENSING SYSTEM AND DISPENSING METHOD**

ABGABESYSTEM UND ABGABEVERFAHREN

SYSTÈME ET PROCÉDÉ DE DISTRIBUTION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.09.2017 JP 2017190413**

(43) Date of publication of application:
**03.04.2019 Bulletin 2019/14**

(73) Proprietors:
• **Kabushiki Kaisha Yaskawa Denki**
  **Kitakyushu-shi, Fukuoka 806-0004 (JP)**
• **Robotic Biology Institute Inc.**
  **Tokyo 135-0064 (JP)**

(72) Inventors:
• **KARIYAZAKI, Hirokazu**
  **Kitakyushu-shi, Fukuoka 806-0004 (JP)**
• **NAGASAKI, Takashi**
  **Kitakyushu-shi, Fukuoka 806-0004 (JP)**

• **KAMEI, Motohisa**
  **Kitakyushu-shi, Fukuoka 806-0004 (JP)**
• **ABE, Noriko**
  **Kitakyushu-shi, Fukuoka 806-0004 (JP)**
• **KIZAKI, Takahiro**
  **Kitakyushu-shi, Fukuoka 806-0004 (JP)**
• **INADA, Kenji**
  **Kitakyushu-shi, Fukuoka 806-0004 (JP)**
• **MATSUKUMA, Kenji**
  **Tokyo, 135-0064 (JP)**

(74) Representative: **Viering, Jentschura & Partner mbB**
  **Patent- und Rechtsanwälte**
  **Am Brauhaus 8**
  **01099 Dresden (DE)**

(56) References cited:
**EP-A2- 0 506 022     WO-A2-2016/191646**
**US-A1- 2003 204 331     US-A1- 2008 072 664**

**Description**

TECHNICAL FIELD

The disclosed embodiment relates to a dispensing system and a dispensing method.

BACKGROUND ART

International publication No. WO 2009/130318 describes a liquid processing work station including a dispensing robot and a control computer. The control computer controls the dispensing robot to perform operations such as absorbing, mixing, or distributing a liquid sample using a pipette.

A known dispensing system is described in US 2008/072664 A1.

DISCLOSURE OF THE INVENTION

Problem to be solved by the Invention

There has been a problem with the prior art that the user is required to select a pipette, select a dispensing position, or determine liquid parameters using input means, which increases the user's work load.

The present invention, which has been made in view of such a problem, is intended to provide a dispensing system and a dispensing method capable of reducing the user's work load.

Means for Solving the Problem

According to the invention, a dispensing system according to claim 1 is provided. Some further designs of the dispensing system are defined by the dependent claims. Moreover, according to the invention, a dispensing method according to claim 4 is provided.

Advantages of the Invention

According to the present invention, it is possible to provide a dispensing system and a dispensing method capable of reducing the user's work load.

BRIEF DESCRIPTION OF THE DRAWINGS

A more complete appreciation of the disclosure and many of the attendant advantages thereof will be readily obtained as the same becomes better understood by reference to the following detailed description when considered in connection with the accompanying drawings, wherein:

FIG. 1 is an explanatory diagram schematically expressing an example of the overall configuration of a dispensing system of an embodiment;
FIG. 2 is an explanatory diagram representing an example of the configuration of a microplate;
FIG. 3 is an explanatory diagram representing an example of the content of a setting file;
FIG. 4 is an explanatory diagram representing an example of the functional configuration of a host controller and a robot controller when diluting stock solution to a target concentration by dispensing diluent to a microplate;
FIG. 5 is an explanatory diagram representing a specific example of a dispensing operation of diluent when diluting the stock solution to the target concentration by dispensing the diluent to the microplate;
FIG. 6 is a flowchart representing an example of the processing steps performed by the host controller and the robot controller when diluting the stock solution to the target concentration by dispensing the diluent to the microplate;
FIG. 7A is an explanatory diagram representing an example of the content of the setting file at the time of acquisition;
FIG. 7B is an explanatory diagram representing an example of the content of the setting file at the time of completion of calculation of a first dispensing amount of diluent;
FIG. 7C is an explanatory diagram representing an example of the content of the setting file at the time of storing the execution result of dispensing the diluent;
FIG. 8 is an explanatory diagram representing an example of the functional configuration of the host controller and the robot controller when extracting a matter from the microplate as much as a target amount of matter by dispensing the stock solution;
FIG. 9 is an explanatory diagram representing a specific example of a dispensing operation of the stock solution when extracting the matter from the microplate as much as the target amount of matter by dispensing the stock solution;
FIG. 10 is a flowchart representing an example of processing steps performed by the host controller and the robot controller when extracting the matter from the microplate as much as the target amount of matter by dispensing stock solution;
FIG. 11A is an explanatory diagram representing an example of the content of the setting file at the time of acquisition;
FIG. 11B is an explanatory diagram representing an example of the content of the setting file at the time of completion of calculation of a second dispensing amount of the stock solution;
FIG. 11C is an explanatory diagram representing an example of the content of the setting file at the time of storing the execution result of dispensing the stock solution; and
FIG. 12 is an explanatory diagram representing an example of the hardware configuration of the controller.

MODE FOR CARRYING OUT THE INVENTION

An embodiment will be described below, referring to the drawings.

<1. Overall configuration of dispensing system>

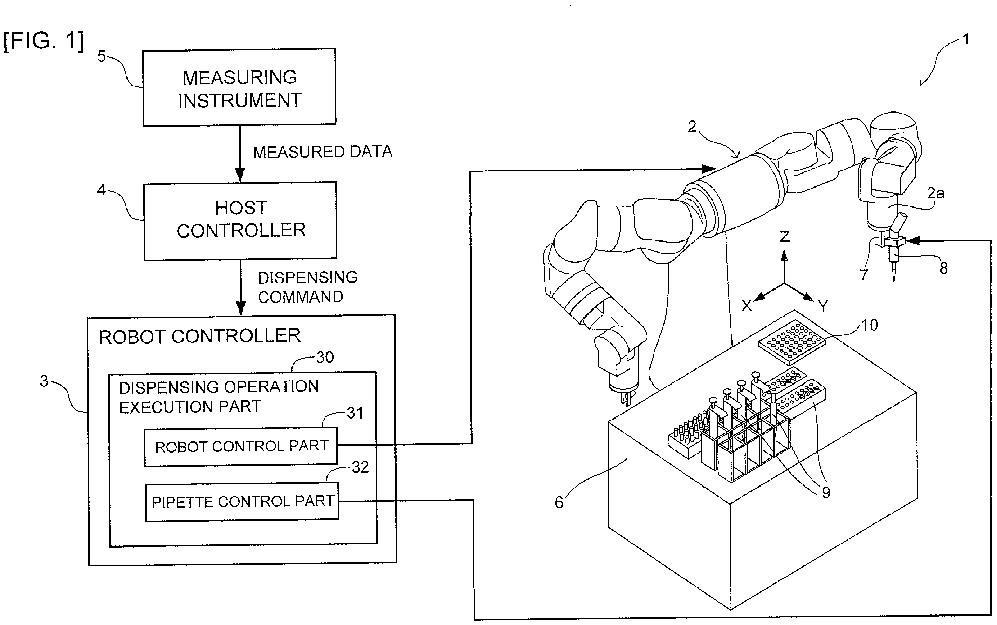

First, a schematic example of the overall configuration of a dispensing system of the present embodiment will be described, referring to FIG. 1.

As illustrated in FIG. 1, a dispensing system 1 having a robot 2, a robot controller 3, and a host controller 4, performs dispensing on the basis of measured data provided by a measuring instrument 5.

The robot 2 (exemplary automatic dispensing machine) performs dispensing work related to the content of the container. In the present embodiment, a case of using a microplate (also referred to as a multi-plate) will be described as an example of the container. The robot 2, which is a double-armed robot having two robot arms, has a gripper-type hand 7 mounted on an arm tip part 2a as an end effector. The hand 7 is gripping an electric pipette 8 (exemplary pipette) to be used for dispensing work. The robot 2 is capable of position control and attitude control of the hand 7 and/or the electric pipette 8 in workspace coordinates XYZ which have been set on the basis of the robot 2 or the like. The robot 2 has a work table 6 disposed around it, with a plurality of dispensing appliances 9 including a microplate 10 being provided on a predetermined position on the top surface of the work table 6.

Not that the robot 2 is not limited to a double-armed robot and may be any other type of robot such as a single-armed vertical multi-joint robot or horizontal multi-joint (scalar) robot including a plurality of joint axes (e.g., six axes). In addition, a unit capable of automatic dispensing may be used in place of a robot. For example, a dispensing dedicated machine may also be used, including an X-direction bar movably disposed in the X-axis direction relative to the microplate 10 and a Y-direction bar movably disposed in the Y-axis direction, with the electric pipette 8 being attached to the Y-direction bar and movably in the Z-direction.

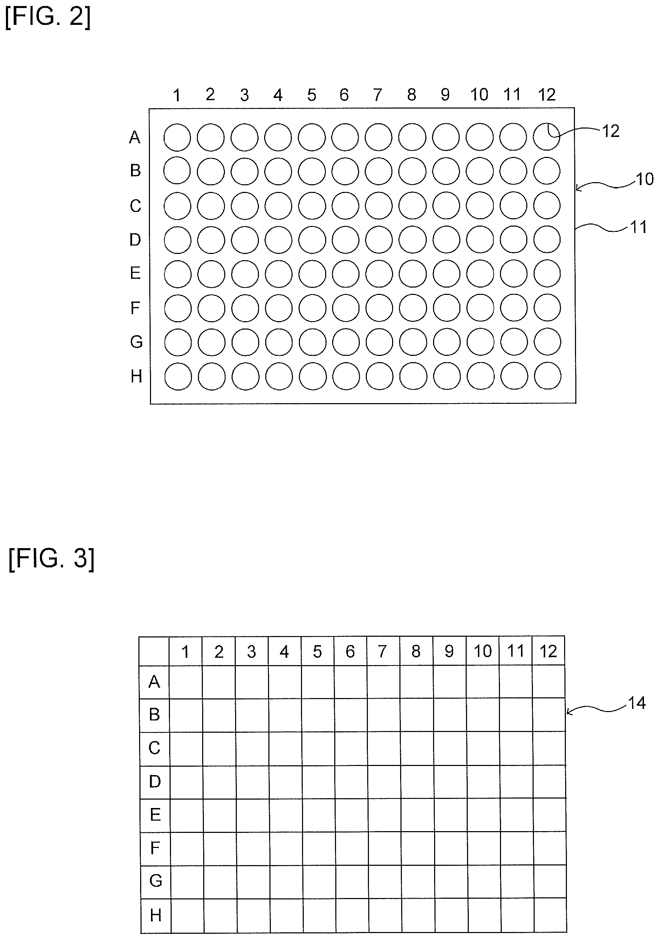

The measuring instrument 5 measures the properties of the content received in the microplate 10. In the example, the measuring instrument 5, which is a spectrophotometer, for example, measures concentration (an exemplary property), for example, as the properties of the content by absorptiometry. Measurement of concentration is performed for each content received in a plurality of wells 12 (an exemplary container and an exemplary receiving part, see FIG. 2 described below) of the microplate 10. Measurement data (concentration data in the example) acquired by the measuring instrument 5 is output to the host controller 4.

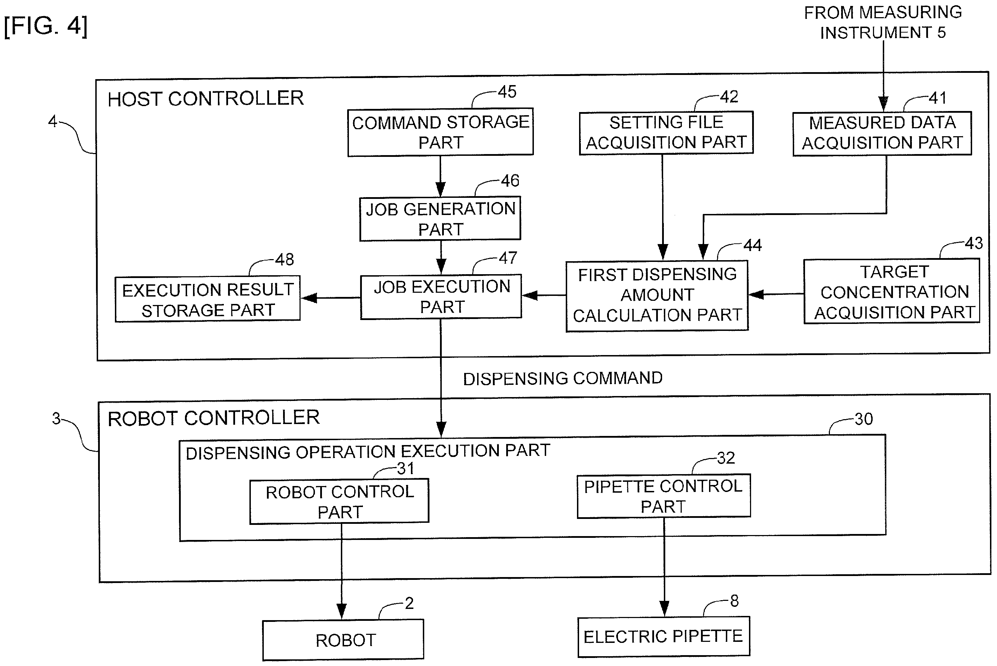

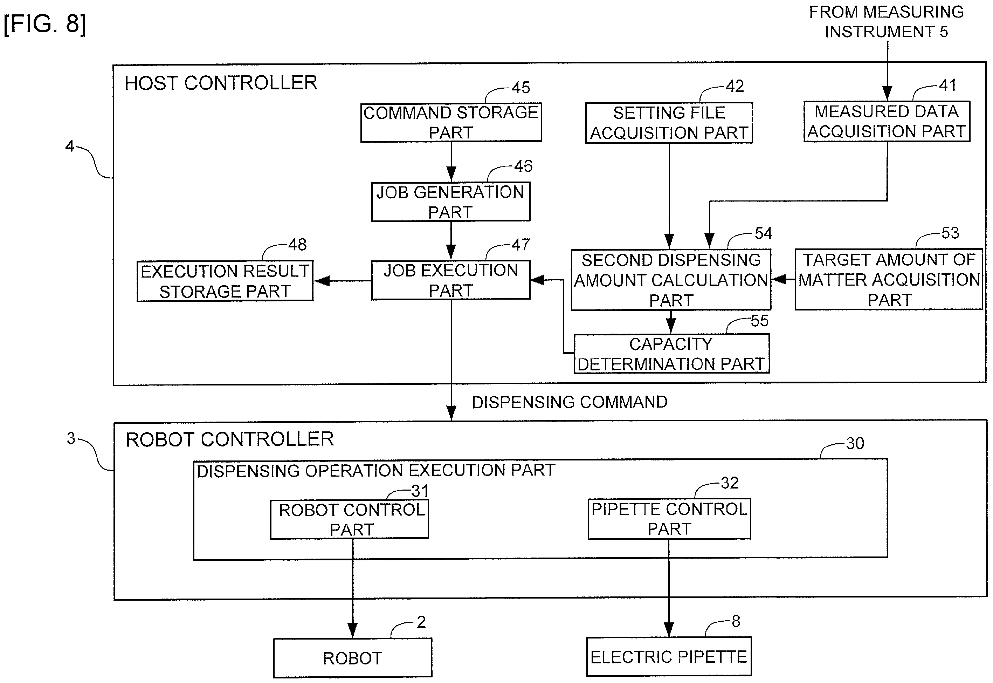

The host controller 4 acquires the measured data or the like, generates a dispensing command, and outputs the dispensing command to the robot controller 3. The host controller 4 is configured as a computer (e.g., personal computer or the like) having, for example, an arithmetic operation unit, a storage unit, an input unit, or the like. Details of the function of the host controller 4 will be described below (see FIGs. 4 and 8 or the like).

The robot controller 3 controls the robot 2 to perform a predetermined dispensing operation on the basis of the dispensing command from the host controller 4. The robot controller 3 includes a dispensing operation execution part 30, and the dispensing operation execution part 30 has a robot control part 31 and a pipette control part 32. Here, the robot controller 3 may be disposed integrally with the robot 2, or may be disposed as a separate body. The robot controller 3 is configured as a computer having, for example, an arithmetic operation unit, a storage unit, an input unit, or the like. In addition, for example, the robot controller 3 may be configured by a combination with a programmable logic controller (PLC). Details of the function of the robot controller 3 will be described below (see FIGs. 4 and 8 or the like).

<2. Example of microplate and setting file>

Next, an example of the microplate and the setting file will be described, referring to FIGs. 2 and 3.

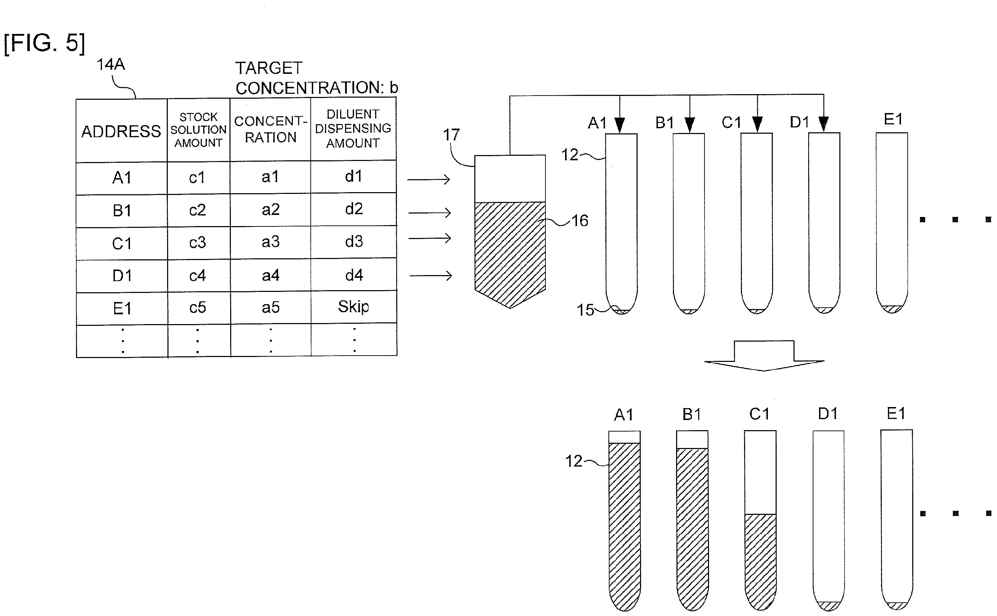

As illustrated in FIG. 2, the microplate 10 (an exemplary reservoir) includes a plurality of wells 12 (an exemplary receiving part), which are bottomed hole parts, on a resin-made board 11, for example. Each of the wells 12 receives stock solution 15 (an exemplary content, see FIG. 5 described below). Here, each one of the wells 12 corresponds to a container. In the example illustrated in FIG. 2, the microplate 10 has a total of 96 of the wells 12, i.e., 8 vertically (row numbers A - H) and 12 laterally (column numbers 1 - 12). The shape of the wells 12 is not limited in particular and may be, for example, cylindrical, prismatic, conic, pyramidal, or the like. In addition, the number of wells is not limited to 96 and may be other numbers (numbers conforming to a standard such as t, for example, 48, 384, etc.). Here, in place of the microplate, an object or the like having, for example, a plurality of tubes, a plurality of test tubes, an eight-column tube disposed on the jig may be used.

FIG. 3 illustrates an example of the content of the setting file. The setting file is an electronic file used for setting data related to the content of each of the wells 12 of the microplate 10. As illustrated in FIG. 3, a setting file 14 has stored therein data related to the content of the wells 12 corresponding to each address specified by a row number and a column number of the wells 12 of the microplate 10. The setting file 14 may be stored in an appropriate storage medium (e.g., ROM, RAM, hard disk, etc.) of the host controller 4, or may be stored in an external storage medium (e.g., optical disk such as CD or DVD, or semiconductor memory, etc.). The setting file 14 is read by the host controller 4, and various data are written therein via user input or automatic processing by software.

<3. Case of diluting stock solution to target concentration by dispensing diluent>

Next, there will be described a case of diluting stock solution to the target concentration by dispensing the diluent to a microplate as an example of dispensing work.

(3-1. Functional configuration of host controller and robot controller)

Referring to FIG. 4, there will be described an example of the functional configuration of the host controller 4 and the robot controller 3 when diluting stock solution to a target concentration by dispensing the diluent to the microplate 10.

As illustrated in FIG. 4, the host controller 4 has a measured data acquisition part 41, a setting file acquisition part 42, a target concentration acquisition part 43, a first dispensing amount calculation part 44, a command storage part 45, a job generation part 46, a job execution part 47, and an execution result storage part 48.

The measured data acquisition part 41 acquires measured data (concentration) of the concentration of the stock solution 15 of each of a plurality of wells 12 of the microplate 10 measured by the measuring instrument 5, from the measuring instrument 5. The acquired measured data is written to a setting file 14A (see FIG. 5 or the like described below).

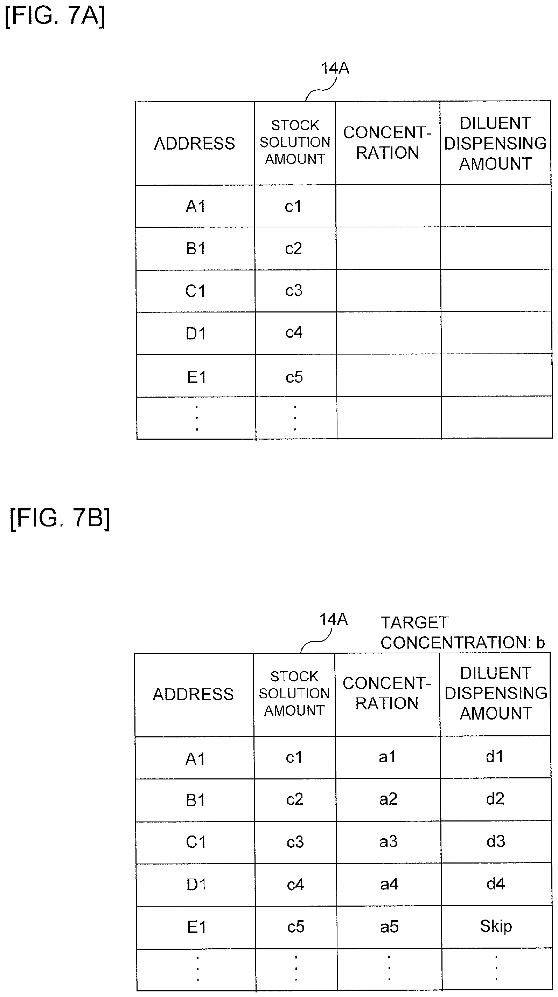

The setting file acquisition part 42 acquires the setting file 14A (see FIG. 5 or the like described below) by reading from an appropriate storage medium of the host controller 4 or reading from an external storage medium. The setting file 14A has a liquid amount of the stock solution 15 of each of the wells 12 preliminarily written therein via user input or the like (see FIG. 7A described below).

The target concentration acquisition part 43 acquires a target concentration to be achieved by dilution of the stock solution 15. In the case that the target concentration is included in a command parameter of a command stored in the command storage part 45, for example, the target concentration acquisition part 43 acquires the target concentration from the command parameter. Alternatively, the target concentration may be set in the setting file 14A in the case that the target concentration has been preliminarily set by user input or the like at the time of creating the setting file 14A. Here, the target concentration may be common to all the wells 12, or may be different for each of the wells 12. The present embodiment describes the case that the target concentration is common to all the wells 12.

The first dispensing amount calculation part 44 calculates, for each of the wells 12, a dispensing amount (referred to "first dispensing amount" below, as appropriate) of diluent 16 (see FIG. 5 described below) required for diluting the stock solution 15 received in each of the wells 12 of the microplate 10 to the target concentration, on the basis of the measured data (concentration) acquired by the measured data acquisition part 41, the amount of stock solution included in the setting file 14A acquired by the setting file acquisition part 42, and the target concentration acquired by the target concentration acquisition part 43. For example, letting c be the liquid amount of the stock solution 15, a be the concentration, b be the target concentration, and d be the first dispensing amount of the diluent 16, the first dispensing amount d is calculated by the following formula:

$$d = a \times (c/b) - c$$

where $a \times c = (c+d) \times b$. The calculated first dispensing amount is written to the setting file 14A (see FIG. 5 or the like described below).

Here, the first dispensing amount is not calculated in the case that it is impossible to calculate the dispensing amount of the diluent 16 required for diluting the stock solution 15 to the target concentration because the stock solution concentration is smaller than the target concentration, for example. In such a case, a phrase "Skip" expressing the failure of calculation, for example, is stored in the dispensing amount field for the corresponding wells 12 in the setting file 14A (see FIG. 5 or the like described below).

In the command storage part 45, various commands and command parameters related to dispensing work such as "add", "mix", "transfer", or the like, to the stock solution 15 in each of the wells 12 of the microplate 10 are stored. The command parameters preliminarily may have the target concentration to be achieved by dilution of the stock solution 15 included therein as described above.

The job generation part 46 combines one of the wells 12 to be subjected to the dispensing work with various commands stored in the command storage part 45 so as to generate a job to each of the wells 12 of the microplate 10, i.e., an command of dispensing work that dilutes the stock solution 15 to the target concentration by dispensing the diluent 16 to the well 12 of interest.

The job execution part 47 outputs dispensing command to the robot controller 3, on the basis of the job for each of the wells 12 generated in the job generation part 46 and the first dispensing amount of the diluent 16 calculated by the first dispensing amount calculation part 44.

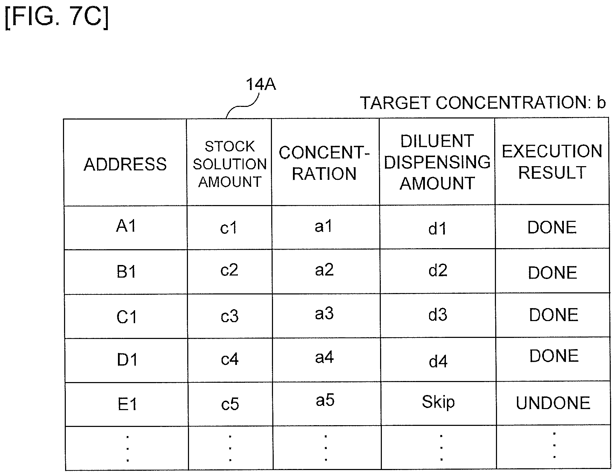

The execution result storage part 48 stores the execution result of executing the dispensing operation in each of the wells 12. Specifically, the dispensing operation of the diluent 16 is performed by the robot 2 in the case that the dispensing amount of the diluent 16 has been calculated by the first dispensing amount calculation part 44. As a result, a phrase "DONE" expressing the completion of execution, for example, is stored in the execution result field of the corresponding well 12 in the setting file 14A (see FIG. 7C described below). On the other hand, the dispensing operation of the diluent 16 is not performed by the robot 2 in the case that the dispensing amount of the diluent 16 has not been calculated by the first dispensing amount calculation part 44. As a result, a phrase "UNDONE" expressing the failure of execution, for example, is stored in the execution result field of the corresponding well 12 in the setting file 14A (see FIG. 7C described below).

The robot controller 3 includes the dispensing operation execution part 30 having the robot control part 31 and the pipette control part 32 as described above. In response to the dispensing command from the job execution part 47 of the host controller 4, the dispensing operation execution part 30 causes the robot control part 31 to control the operation of the robot 2 and causes the pipette control part 32 to control the operation of the electric pipette 8. Accordingly, the dispensing operation execution part 30 controls the robot 2 to perform a dispensing operation that dilutes the stock solution 15 of each of the wells 12 to the target concentration by dispensing the diluent 16 into each of the wells 12 of the microplate 10.

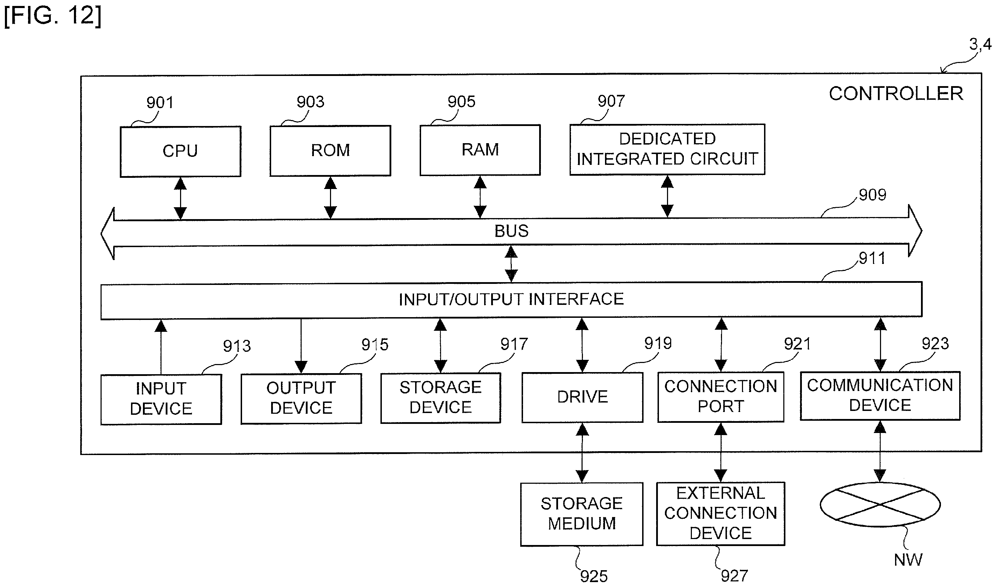

Here, the processing by the measured data acquisition part 41 and the first dispensing amount calculation part 44 of the host controller 4, and the processing by the dispensing operation execution part 30 of the robot controller 3 or the like are not limited to the allocation example of such processing. For example, the processing may be performed by a smaller number of processing parts (e.g., a single processing part), or may be performed by further subdivided processing parts. In addition, respective functions of the host controller 4, and functions other than that of the part supplying driving power to the robot 2 in the robot controller 3 (inverter, etc.) may be implemented by a program executed by a CPU 901 (see FIG. 12) described below. In addition, a part or all of the functions may be implemented by an actual device such as an ASIC, an FPGA, or other electric circuits.

(3-2. Specific example of dispensing operation of diluent)

Next, referring to FIG. 5, there will be described a specific example of dispensing operation of diluent performed by a robot when diluting stock solution to the target concentration by dispensing the diluent to the microplate.

In FIG. 5, the setting file 14A has written therein the amounts of the stock solution 15 received in the wells 12 respectively addressed A1, B1, C1, D1, E1,..., concentration of the stock solution 15 acquired by the measured data acquisition part 41, and a first dispensing amount of the diluent 16 calculated by the first dispensing amount calculation part 44. In addition, a tube 17 having the diluent 16 received therein, for example, is prepared on the work table 6 as a dispensing appliance 9.

In the example illustrated in FIG. 5, the target concentration acquired by the target concentration acquisition part 43 is b. For the well 12 addressed A1, as indicated by the setting file 14A, the amount of the stock solution 15 is c1, the concentration is 1a1, and the dispensing amount of the diluent 16 is d1. For the well 12 addressed B1, the amount of the stock solution 15 is c2, the concentration is a2, and dispensing amount of the diluent 16 is d2. For the well 12 addressed C1, the amount of the stock solution 15 is c3, the concentration is a3, and the dispensing amount of the diluent 16 is d3. For the well 12 addressed D1, the amount of the stock solution 15 is c4, the concentration is a4, and the dispensing amount of the diluent 16 is d4. For the well 12 addressed E1, the amount of the stock solution 15 is c5 and the concentration is a5. Here, for the well 12 addressed E1, for example the stock solution concentration a5 is lower than the target concentration b, whereby it is impossible to dilute the stock solution 15 to the target concentration by dispensing the diluent 16, and therefore the dispensing amount field of the diluent 16 is set to "Skip".

First, with the control of the robot control part 31, the robot 2 transfers the electric pipette 8 gripped by the hand 7 to the upper part of the tube 17. The robot 2 then lowers the electric pipette 8 to dip the pipette tip into the diluent 16 in the tube 17, and keeps it in that state. Subsequently, control of the pipette control part 32, the electric pipette 8 sets the absorption amount of the diluent 16 to the dispensing amount d1 for the well 12 addressed A1, for example, and then raises the piston to absorb the diluent 16 as much as the dispensing amount. Subsequently, control of the robot control part 31, the robot 2 uses the hand 7 to transfer the electric pipette 8 above the well 12 addressed A1, and then lowers the electric pipette 8 to insert the pipette tip into the upper part of the well 12. Then, with the control of the pipette control part 32, the electric pipette 8 lowers the piston to discharge the diluent 16 from the electric pipette 8 into the well 12 to perform dispensing into the well 12. Accordingly, the stock solution 15 in the well 12 addressed A1 is diluted by the diluent 16 to reach the target concentration.

Next, with the control of the robot 2 by the robot control part 31 and control of the electric pipette 8 by the pipette control part 32, the robot 2 transfers the electric pipette 8 to the tube 17, and absorbs the diluent 16 as much as the dispensing amount d2 for the well 12 addressed B1, for example, which is the next target of dispensing. Subsequently, the robot 2 transfers the electric pipette 8 to the well 12 addressed B1, and dispenses the diluent 16 into the well 12 so that the stock solution 15 is diluted to the target concentration.

Such a dispensing operation of the diluent 16 to the wells 12 by the robot 2 is performed for each of a plurality of wells 12 addressed A1 - H12 of the microplate 10, and the series of dispensing work are completed. Here, dispensing operation by the robot 2 is not performed for the well 12 (addressed E1 in the example of FIG. 5) for which the dispensing amount has not been calculated.

(3-3. Processing steps performed by host controller and robot controller)

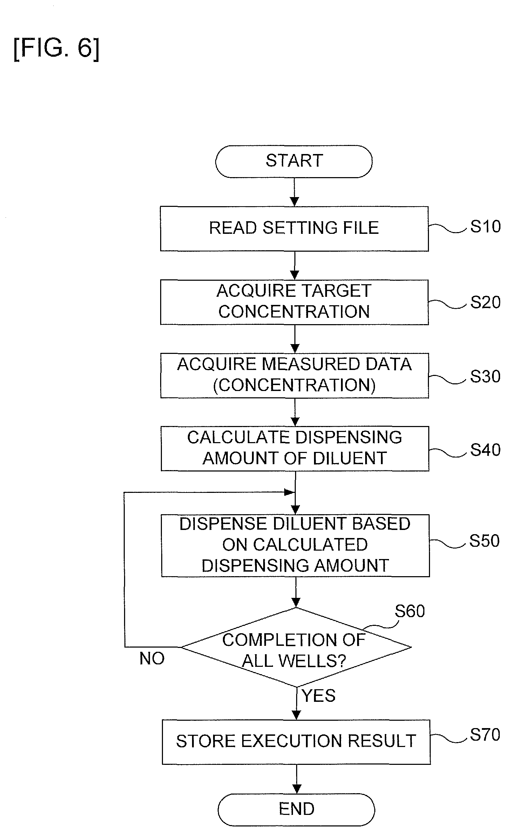

Next, referring to FIG. 6, there will be described an example of the processing steps by the host controller 4 and the robot controller 3 when diluting the stock solution to the target concentration by dispensing the diluent to the microplate.

At step S10, the host controller 4 causes the setting file acquisition part 42 to read and to acquire the setting file 14A. The content of the setting file 14A at the time of acquisition is as illustrated in FIG. 7A, for example, having the address of well 12 of the microplate 10 and the amount of the stock solution 15 received in the well 12 written therein. Here, the concentration of the stock solution 15 and the first dispensing amount of the diluent 16 are left blank.

At step S20, the host controller 4 causes the target concentration acquisition part 43 to acquire, for example from the command parameters, the target concentration to be achieved by dilution of the stock solution 15 of each of the wells 12 of the microplate 10. Here, the target concentration may be set according to the setting file 14A as described above. In such a case, the present step is unnecessary because the target concentration has been written in the setting file 14A acquired at step S10.

At step S30, the host controller 4 causes the measured data acquisition part 41 to acquire the measured data of the concentration of the stock solution 15 of each of the wells 12 of the microplate 10 measured by the measuring instrument 5.

At step S40, the host controller 4 causes the first dispensing amount calculation part 44 to calculate, for each of the wells 12, the first dispensing amount required for diluting the stock solution 15 of the wells 12 to the target concentration, on the basis of the measured data (concentration) acquired at step S30, the amount of stock solution included in the setting file 14A acquired at step S10, and the target concentration acquired at step S20. The setting file 14A at the time of completion of calculation of the dispensing amount of the diluent 16 is as illustrated in FIG. 7B, for example, having the target concentration, the concentration of the stock solution 15, and the dispensing amount of the diluent 16 written therein. Here, the host controller 4 outputs a dispensing command from the job execution part 47 to the robot controller 3 at the time of completion of calculation of the first dispensing amount.

At step S50, the robot controller 3, on the basis of the dispensing command from the job execution part 47, controls the robot 2 to perform the work of dispensing the diluent 16 on the basis of the dispensing amount calculated at step S40, with the control of the robot 2 by the robot control part 31 of the dispensing operation execution part 30 and the control of the electric pipette 8 by the pipette control part 32.

At step S60, the robot controller 3 determines whether or not dispensing of the diluent 16 into all the wells 12 of the microplate 10 has been completed. In the case that there remains a well 12 to which dispensing of the diluent 16 has not been performed (NO at Step S60), the process flow returns to step S50 and repeats the similar processing. Alternatively, in the case that dispensing of the diluent 16 into all the wells 12 has been completed (YES at Step S60), the process flow proceeds to step S70.

At step S70, the host controller 4 causes the execution result storage part 48 to store the execution result of dispensing. The setting file 14A at the time of storing the execution result of dispensing is as illustrated in FIG. 7 C, for example, with either a phrase "DONE" expressing the success of execution or "UNDONE" expressing the failure of execution being written in the execution result field. Subsequently, the flow is terminated.

Here, the processing steps are only an example, and at least a part of the steps may be deleted or changed, or steps other than those described above may be added.

<4. Case of extracting matter as much as the target amount of matter by dispensing stock solution >

Next, there will be described a case of extracting matter as much as the target amount of matter from a well of the microplate by dispensing stock solution, according to the claimed invention.

(4-1. Functional configuration of host controller and robot controller)

Referring to FIG. 8, there will be described an example of the functional configuration of the host controller 4 and the robot controller 3 when extracting a matter as much as a target amount of matter from the wells of the microplate 10 by dispensing stock solution, according to the claimed invention. Here, components in FIG. 8 that are similar to those of FIG. 4 described above are provided with identical reference numerals, with explanation thereof being omitted as appropriate.

As illustrated in FIG. 8, the host controller 4 has a target amount of matter acquisition part 53, a second dispensing amount calculation part 54, and a capacity determination part 55, besides the above-mentioned measured data acquisition part 41, the setting file acquisition part 42, the command storage part 45, the job generation part 46, the job execution part 47, and the execution result storage part 48.

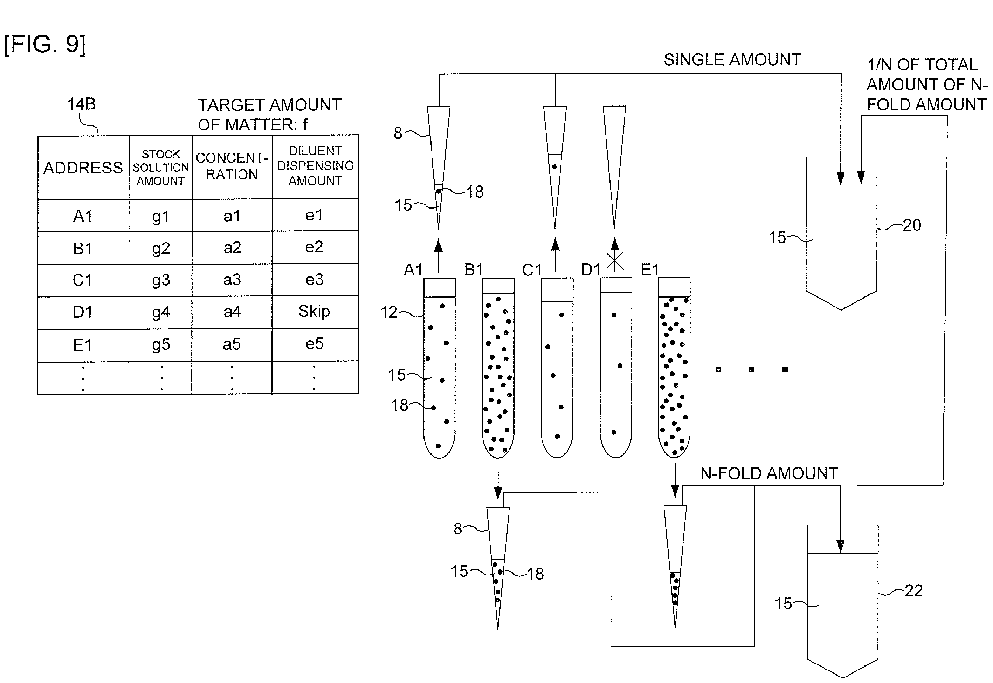

The target amount of matter acquisition part 53 acquires, with regard to a matter 18 included in the stock solution 15, a target amount of matter to be extracted by dispensing the stock solution 15. The matter 18 is a matter forming a living body such as, for example, DNA, RNA, amino acid, protein, polysaccharide or biological cells. In addition, the target amount of matter is defined by the number of component particles (atoms, molecules, ions, electrons or other particles, or aggregation of such particles) forming the matter, or a quotient (mol) resulted from dividing the number by the Avogadro constant, or alternatively the mass or the like of the matter, for example. In the case that a target amount of matter is included in a command parameter of a command stored in the command storage part 45, for example, the target amount of matter acquisition part 53 acquires the target amount of matter from the command parameter. Alternatively, in the case that the target amount of matter has been preliminarily set by user input or the like at the time of creating the setting file 14B (see FIG. 9 or the like described below), the target amount of matter may be set in the setting file 14B. Here, the target amount of matter may be common to all the wells 12, or may be different for each of the wells 12. The present embodiment describes the case that the target amount of matter is common to all the wells 12.

The second dispensing amount calculation part 54 calculates, for each of the wells 12, a dispensing amount (referred to "second dispensing amount" below, as appropriate) of the stock solution 15 required for extracting the matter 18 as much as the target amount of matter from each of the wells 12 of the microplate 10, on the basis of the measured data (the concentration) acquired by the measured data acquisition part 41, the amount of stock solution included in the setting file 14B acquired by the setting file acquisition part 42, and the target amount of matter acquired by the target amount of matter acquisition part 53. For example, letting a be the concentration of stock solution, e be the second dispensing amount of the stock solution, and f be the target amount of matter, the second dispensing amount e is calculated by the following formula:

$$e = f/a$$

where $f = a \times e$. The calculated second dispensing amount is written to the setting file 14B (see FIG. 9 described below).

Here, in the case that the amount of the stock solution 15 does not reach the second dispensing amount, for example, it is impossible to dispense the stock solution 15 for extracting the matter 18 as much as the target amount of matter, and therefore the second dispensing amount is not calculated. In such a case, a phrase "Skip" expressing the failure of calculation, for example, is stored in the dispensing amount field of the corresponding well 12 in the setting file 14B (see FIG. 9 or the like described below).

The capacity determination part 55 determines whether or not the second dispensing amount of the stock solution 15 calculated by the second dispensing amount calculation part 54 falls within the capacity range of the electric pipette 8.

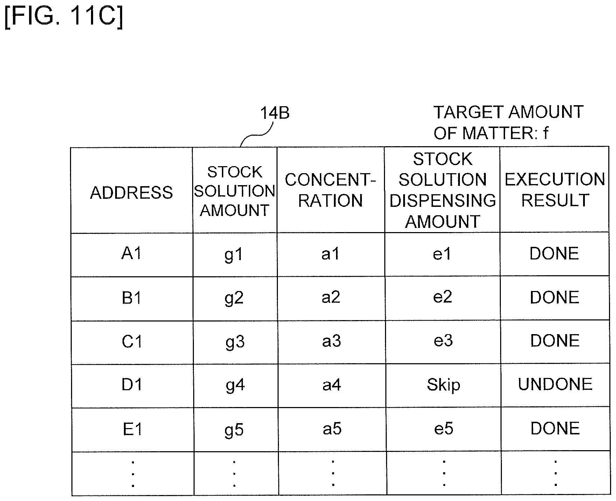

The execution result storage part 48 stores the execution result of executing the dispensing operation in each of the wells 12. Specifically, in the case that the second dispensing amount of the stock solution 15 has been calculated by the second dispensing amount calculation part 54, dispensing operation of the stock solution 15 is performed by the robot 2, and therefore a phrase "DONE" expressing the completion of execution, for example, is stored in the execution result field of the corresponding well 12 in the setting file 14B (see FIG. 11C described below). On the other hand, in the case that the second dispensing amount of the stock solution 15 has not been calculated by the second dispensing amount calculation part 54, dispensing operation of the stock solution 15 is not performed by the robot 2, and therefore a phrase "UNDONE" expressing the failure of execution, for example, is stored in the execution result field of the corresponding well 12 in the setting file 14B (see FIG. 11C described below).

The dispensing operation execution part 30 of the robot controller 3 controls the robot 2 to perform a dispensing operation of the stock solution 15. On this occasion, the dispensing operation execution part 30 changes the destination of dispensing according to whether the second dispensing amount falls within, or falls outside, the capacity range of the electric pipette 8, on the basis of the execution result of determination by the capacity determination part 55. Specifically, in the case that the second dispensing amount falls within the capacity range of the electric pipette 8, the dispensing operation execution part 30 dispenses the stock solution 15 into a first tube 20 (single-amount container, see FIG. 9 described below) as much as the second dispensing amount, which is the original dispensing amount (single amount). Alternatively, in the case that the second dispensing amount falls outside the capacity range of the electric pipette 8 (the minimum value in the capacity range in which the electric pipette 8 can perform absorption is larger than the second dispensing amount), an N-fold amount by multiplying the second dispensing amount is set by a predetermined value N, and the dispensing operation execution part 30 dispenses the stock solution 15 into a second tube 22 (N-fold amount container, see FIG. 9 described below described below) as much as the N-fold amount of the second dispensing amount. Subsequently, the dispensing operation execution part 30 dispenses as much as 1/N of the total amount of the N-fold amount stock solution into the first tube 20 again from the second tube 22 having collected therein the N-fold amount of the stock solution 15, and adds the resulted solution to the total amount of stock solution which has been dispensed into the first tube 20 in terms of single-fold amount. Here, the value of N is not limited in particular, and any multiple maybe used as long as the N-fold amount of dispensing falls within the capacity range of the electric pipette 8.

The processing by the second dispensing amount calculation part 54 and the capacity determination part 55 of the host controller 4 or the like are not limited to the allocation example of such processing. For example, the processing may be performed by a smaller number of processing parts (e.g., a single processing part), or may be performed by further subdivided processing parts. In addition, respective functions of the host controller 4 may be implemented by a program executed by a CPU 901 (see FIG. 12) described below. In addition, a part or all of the functions may be implemented by an actual device such as an ASIC, an FPGA, or other electric circuits.

(4-2. Specific example of dispensing operation of stock solution)

Next, referring to FIG. 9, there will be described a specific example of dispensing operation of stock solution performed by a robot when extracting a matter from a well as much as a target amount of matter by dispensing stock solution, according to the claimed invention.

In FIG. 9, the setting file 14B has written therein the amounts of the stock solution 15 received in the wells 12 respectively addressed A1, B1, C1, D1, E1,..., concentration of the stock solution 15 acquired by the measured data acquisition part 41, and a second dispensing amount of the stock solution 15 calculated by the second dispensing amount calculation part 54. In addition, the first tube 20 and the second tube 22, for example, are prepared on the work table 6 as the dispensing appliance 9.

In the example illustrated in FIG. 9, the target amount of matter acquired by the target amount of matter acquisition part 53 is f. For the well 12 addressed A1, as indicated by the setting file 14B, the amount of the stock solution 15 is g1, the concentration is a1, and the dispensing amount of the stock solution 15 is e1. For the well 12 addressed B1, the amount of the stock solution 15 is g2, the concentration is a2, and the dispensing amount of the stock solution 15 is e2. For the well 12 addressed C1, the amount of the stock solution 15 is g3, the concentration is a3, and the dispensing amount of the stock solution 15 is e3. For the well 12 addressed D1, the amount of the stock solution 15 is g4 and the concentration is a4. Here, for the well 12 addressed D1, for example the amount g4 of the stock solution 15 is less than the second dispensing amount ($e4 = f/a4$) required for extracting a matter as much as the target amount of matter, whereby it is impossible to extract a matter as much as the target amount of matter by dispensing the stock solution 15, and therefore the field of the stock solution dispensing amount is set to "Skip". For the well 12 addressed E1, the amount of the stock solution 15 is g5, the concentration is a5 and the dispensing amount of the stock solution 15 is e5. Correspondingly, the capacity range in which the electric pipette 8 can perform absorption is Hmin to Hmax, for example.

First, with the control by the robot control part 31, the robot 2 transfers the electric pipette 8 gripped by the hand 7 to the upper part of well 12 addressed A1, for example, which is the target of dispensing. The robot 2 then lowers the electric pipette 8 to dip the pipette tip into the stock solution 15 in the well 12, and holds it in that state. The second dispensing amount of the well 12 addressed A1 is e1, which is within the capacity range (Hmin < e1 < Hmax) of the electric pipette 8. Therefore, with the help of control by the pipette control part 32, the electric pipette 8 sets the absorption amount of the stock solution 15 to the second dispensing amount e1 which is a single amount, and then raises the piston to absorb the stock solution 15 as much as the single-amount dispensing amount, and extracts the matter 18 from the well 12 as much as the target amount of matter f. Subsequently, with the control by the robot control part 31, the robot 2 uses the hand 7 to transfer the electric pipette 8 above the first tube 20, and lowers the electric pipette 8 to insert the pipette tip into the first tube 20. Then, with the help of control by the pipette control part 32, the electric pipette 8 lowers the piston to discharge the single-amount stock solution 15 into the first tube 20 so as to dispense the single-amount stock solution 15 of the well 12 addressed A1 into the first tube 20.

Next, with the control of the robot 2 by the robot control part 31 and control of the electric pipette 8 by the pipette control part 32, the robot 2 transfers the electric pipette 8 to well 12 addressed B1, for example, which is the next target of dispensing. The second dispensing amount of the well 12 addressed B1 is e2, which is outside the capacity range of the electric pipette 8 (e2 < Hmin). Therefore, the electric pipette 8 defines an N-fold amount ($N \times e2$) of the second dispensing amount, sets it as the absorption amount of the electric pipette 8, absorbs the stock solution 15 from the well 12 as much as the N-fold amount, and extracts the matter 18 as much as the N-fold amount of the target amount of matter F. Subsequently, the robot 2 transfers the electric pipette 8 to the second tube 22, and performs dispensing by discharging the N-fold amount of the stock solution 15 into the second tube 22. Accordingly, the N-fold amount of the stock solution 15 of the well 12 addressed B1 is temporarily reserved in the second tube 22.

The dispensing operation of a single-amount of the second dispensing amount of the stock solution 15 from the well 12 into the first tube 20 and the dispensing operation of an N-fold amount of the second dispensing amount of the stock solution 15 from the well 12 into the second tube 22 by the robot 2 in such a manner are performed for each of the plurality of wells 12 addressed A1 - H12 of the microplate 10. In the example illustrated in FIG. 9, the second dispensing amount of the well 12 addressed C1 is e3, which falls within the capacity range of the electric pipette 8 (Hmin < e3 < Hmax), and therefore a single amount is dispensed to the first tube 20. In addition, the second dispensing amount of the well 12 addressed E1 is e5, which is outside the capacity range of the electric pipette 8 (e5 < Hmin), and therefore an n-fold amount ($N \times e5$) is dispensed to the second tube 22. Here, for the well 12 addressed D1, for example, the amount g4 of the stock solution 15 is less than the second dispensing amount (e4 = f/a4), whereby it is impossible to perform dispensing of the stock solution 15 for extracting the matter 18 as much as the target amount of matter, and therefore the second dispensing amount has not been calculated and the dispensing operation by the robot 2 is not performed.

Subsequently, when dispensing of the stock solution 15 into the first tube 20 and dispensing of the stock solution 15 into the second tube 22 are completed for all the wells 12 of the microplate 10, a 1/N amount of the total amount of liquid of the second tube 22 having reserved therein the stock solution 15 in terms of N-fold amount is dispensed into the first tube 20 again by the electric pipette 8 at one time or in several times. Accordingly, it turns out that the first tube 20 includes the matter 18 extracted in terms of the target amount of matter from all the wells 12 of the microplate 10 (except for the wells 12 from which dispensing has not been performed).

(4-3. Processing steps performed by host controller and robot controller)

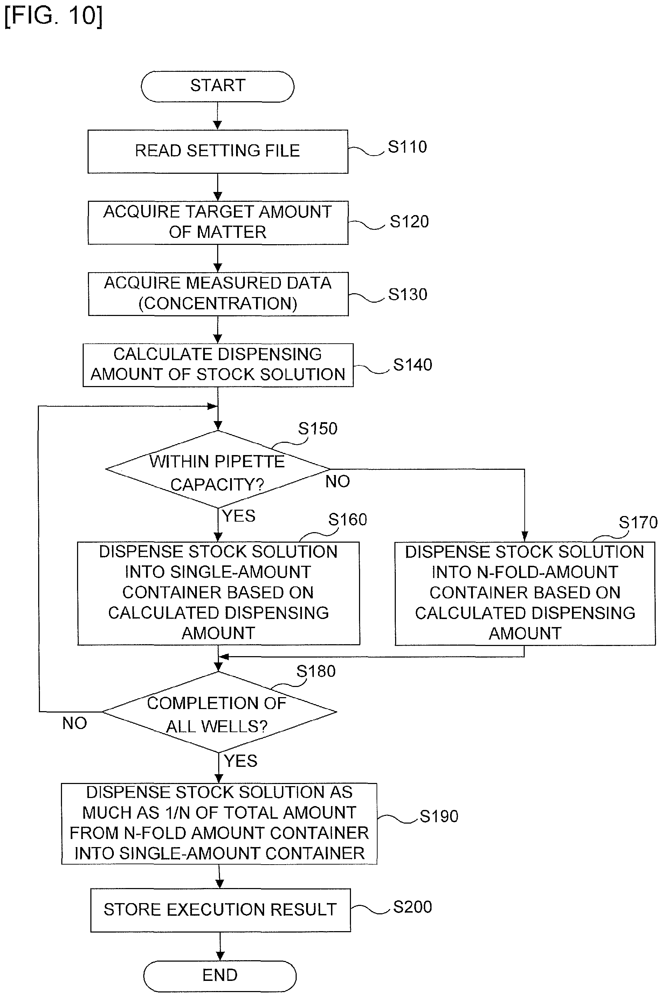

Next, referring to FIG. 10, there will be described an example of processing steps performed by the host controller 4 and the robot controller 3 when extracting the matter from the wells as much as the target amount of matter by the dispensing operation of the stock solution, according to the claimed invention.

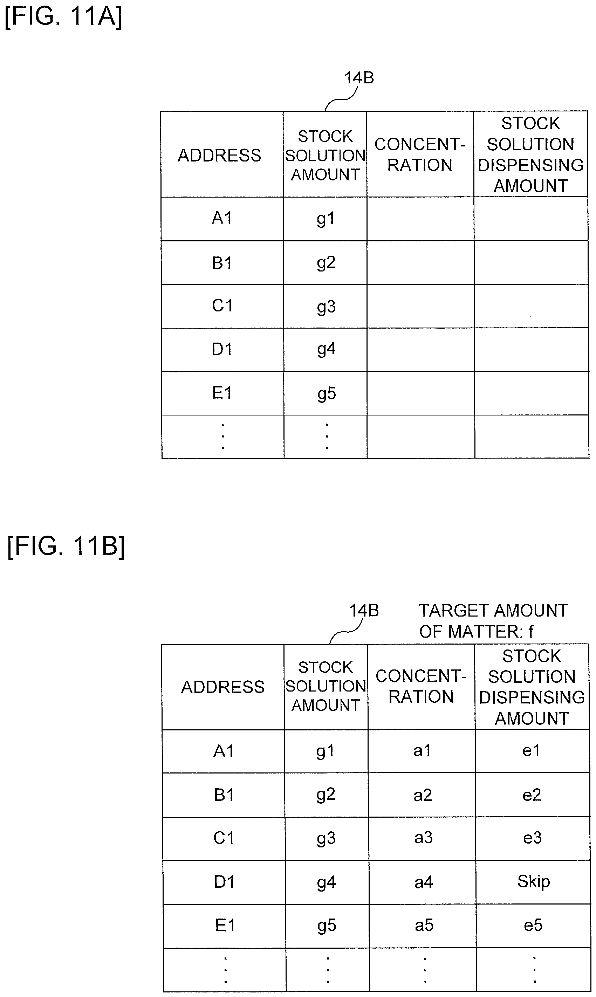

First, at step S110, the host controller 4 causes the setting file acquisition part 42 to read and acquire the setting file 14B. The content of the setting file 14B at the time of acquisition is as illustrated in FIG. 11A, for example, having the address of well 12 of the microplate 10 and the amount of the stock solution 15 received in the well 12 written therein. Here, the concentration of the stock solution 15 and the second dispensing amount of the stock solution 15 are left blank. Upon completion of step S110, the process flow proceeds to step S120.

At step S120, the host controller 4 causes a target amount of matter acquisition part 53 to acquire, for example from the command parameters, the target amount of the matter 18 to be extracted from the stock solution 15 of each of the wells 12 of the microplate 10. Here, the target amount of matter may be set according to the setting file 14B as described above. In such a case, the present step is unnecessary because the target amount of matter has been written in the setting file 14B acquired at step S110.

At step S130, the host controller 4 causes the measured data acquisition part 41 to acquire the measured data of the concentration of the stock solution 15 of each of the wells 12 of the microplate 10 measured by the measuring instrument 5.

At step S140, the host controller 4 causes the second dispensing amount calculation part 54 to calculate, for each of the wells 12, the second dispensing amount of the stock solution 15 required for extracting the matter 18 as much as the target amount of matter from the well 12, on the basis of the measured data (concentration) acquired at step S130 and the target amount of matter acquired at step S120. The setting file 14B at the time of completion of calculation of the second dispensing amount of the stock solution 15 is as illustrated in FIG. 11B, for example, having the target amount of matter, the concentration of the stock solution 15, and the second dispensing amount of the stock solution 15 written therein.

At step S150, the host controller 4 causes the capacity determination part 55 to determine whether or not the second dispensing amount of the stock solution 15 calculated at step S140 falls within the capacity range of the electric pipette 8. In the case that the second dispensing amount falls within the capacity range of the electric pipette 8 (YES at S150), the process flow proceeds to step S160 after having output a corresponding dispensing command to the robot controller 3 from the job execution part 47. Alternatively, in the case that the second dispensing amount falls outside the capacity range of the electric pipette 8 (NO at S150), the process flow proceeds to step S170 after having output a corresponding dispensing command to the robot controller 3 from the job execution part 47.

At step S160, the robot controller 3, on the basis of the dispensing command from the job execution part 47, controls the robot 2 to perform the work of dispensing a single amount of the stock solution 15 into the first tube 20 on the basis of the dispensing amount calculated at step S140, with the control of the robot 2 by the robot control part 31 of the dispensing operation execution part 30 and control of the electric pipette 8 by the pipette control part 32.

At step S170, the robot controller 3, in response to the dispensing command from the job execution part 47, controls the robot 2 to perform the work of dispensing an N-fold amount of the stock solution 15 into the second tube 22 on the basis of the dispensing amount calculated at step S140.

At step S180, the robot controller 3 determines whether or not dispensing of the stock solution 15 into all the wells 12 of the microplate 10 has been completed. In the case that there remains a well 12 to which dispensing of the stock solution 15 has not been performed (NO at Step S180), the process flow returns to step S150 and repeats the similar processing. Alternatively, in the case that dispensing of the stock solution 15 into all the wells 12 has been completed (YES at Step S180), the process flow proceeds to step S190.

At step S190, the robot controller 3 dispenses, from the N-fold amount second tube 22, the stock solution as much as 1/N of the total amount of the second tube 22 into the single-amount first tube 20.

At step S200, the host controller 4 causes the execution result storage part 48 to store the execution result of dispensing. The setting file 14B at the time of storing the execution result of dispensing is as illustrated in FIG. 11C, with either a phrase "DONE" expressing the success of execution or "UNDONE" expressing the failure of execution being written in the execution result field. Subsequently, the flow is terminated.

Here, the processing steps are only an example, and at least a part of the steps may be deleted or changed, or steps other than those described above may be added, as long as the processing steps defined by the claims are present.

<5. Effects of embodiments>

As has been described above, the dispensing system 1 of the present embodiment has the robot 2 configured to perform a dispensing operation related to the stock solution 15 of the microplate 10, the measured data acquisition part 41 configured to acquire measured data of the property of the stock solution 15, and the dispensing operation execution part 30 configured to control the robot 2 to perform a dispensing operation on the basis of the measured data. Accordingly, the following effects are exhibited.

Specifically, in the present embodiment, the measured data is acquired from the measuring instrument 5 configured to measure the property of the stock solution 15, and the robot 2 automatically performs a dispensing operation in accordance with the property of the stock solution 15 on the basis of the measured data. Accordingly, it becomes possible to perform a fine-tuned dispensing operation corresponding to the property of the stock solution 15 by the robot 2, and also to omit the user's labor of inputting the property data of the stock solution 15, whereby the user's work load can be reduced.

In addition, particularly in the present embodiment, the measured data acquisition part 41 acquires the measured data of concentration of the stock solution 15, and the dispensing operation execution part 30 controls the robot 2 to perform a dispensing operation on the basis of the measured data of concentration.

Accordingly, it becomes possible to perform a fine-tuned dispensing operation corresponding to the concentration of the stock solution 15 by the robot 2, and also omit the user's labor of inputting the concentration data of the stock solution 15, whereby the user's work load can be reduced.

In addition, particular in the present embodiment, the dispensing system 1 further has the target concentration acquisition part 43 configured to acquire the target concentration of the stock solution 15, and the first dispensing amount calculation part 44 configured to calculate the first dispensing amount of the diluent 16 into the microplate 10 so that the stock solution 15 reaches the target concentration, on the basis of the measured data and the target concentration, and the dispensing operation execution part 30 controls the robot 2 to perform a dispensing operation that dispenses the first dispensing amount of the diluent 16 into the microplate 10.

Accordingly, it becomes possible to automate the calculation of the dispensing amount and the dispensing operation in order to dilute the stock solution 15 of the microplate 10 to the predetermined target concentration, whereby the user's convenience may be improved.

In the claimed invention, the dispensing system 1 further has a target amount of matter acquisition part 53 configured to acquire the target amount of the matter 18 included in the stock solution 15, and the second dispensing amount calculation part 54 configured to calculate the second dispensing amount of the stock solution 15 so that the matter 18 as much as the target amount of matter is extracted from the microplate 10 on the basis of the measured data and the target amount of matter, and the dispensing operation execution part 30 controls the robot 2 to perform a dispensing operation that dispenses the stock solution 15 of the second dispensing amount into other containers 20 and 22 (the first tube 20 and the second tube 22) from the microplate 10.

Accordingly, it becomes possible to automate the calculation of the dispensing amount and the dispensing operation in order to extract (sample) the matter 18 as much as the target amount of matter from the microplate 10, whereby the user's convenience may be improved.

In the claimed invention, the other containers 20 and 22 include the first tube 20 and the second tube 22, and the dispensing operation execution part 30 controls the robot 2 to perform a dispensing operation that dispenses the stock solution 15 separately into each of the first tube 20 and the second tube 22.

Accordingly, it is possible to select dispensing destinations in accordance with the property of the stock solution 15 or the like, whereby the user's convenience may be further improved.

In the claimed invention, the robot 2 includes the electric pipette 8, the dispensing system 1 further has the capacity determination part 55 configured to determine whether or not the second dispensing amount falls within the capacity range of the electric pipette 8, and the dispensing operation execution part 30 controls the robot 2 to perform a dispensing operation that dispenses the stock solution 15 separately into each of the first tube 20 and the second tube 22 depending on whether the second dispensing amount falls within, or falls outside, the capacity range of the electric pipette 8. Accordingly, the following effect is exhibited.

Specifically, depending on the concentration of the stock solution 15, there is a possibility that the calculated second dispensing amount may fall outside the range of the pipette capacity of the electric pipette 8 and, in such a case, the dispensing operation may not be performed with a good precision.

In the claimed invention, the dispensing destination is divided depending on whether the second dispensing amount falls within, or falls outside, the capacity range of the electric pipette 8. Accordingly, in the case that the second dispensing amount falls outside the capacity range, it is possible to set a predetermined dispensing amount (an N-fold amount resulting from multiplying the second dispensing amount by a predetermined multiplier N) that falls within the capacity range, so that dispensing, from the second tube 22 having collected therein the N-fold amount, the reciprocal amount of the multiplier (1/N) relative to the total amount of the second tube 22 into the first tube 20 having collected therein the single amount of the second dispensing amount may bring about a same dispensing result as the case of collecting a single amount of the second dispensing amount from all the wells 12. Therefore, it is possible to perform a dispensing operation with a good precision regardless of the concentration of the stock solution 15.

In the claimed invention, the container that receives the stock solution 15 is each of the wells 12 in the microplate 10 including the plurality of the wells 12. Accordingly, the following effect is exhibited.

Specifically, when performing a dispensing operation of the stock solution 15 in each receiving part of a reservoir including a large number of receiving parts such as for example, each of the wells 12 of the microplate 10 or each tube in an eight-column tube jig, it is very troublesome for the user to input property data of each the stock solution 15.

According to the claimed invention, the measured data of each of the stock solution 15 is collectively acquired from the measuring instrument 5 and the robot 2 automatically performs a dispensing operation in accordance with the property of each stock solution on the basis of the measured data, whereby it is possible to largely reduce the user's work load.

In addition, particularly in the present embodiment, the dispensing system 1 has the execution result storage part 48 configured to store the execution result of the dispensing operation for each the plurality of the wells 12.

Accordingly, it becomes possible to display or to output the execution result of the dispensing operation in a desired data format, whereby the user's convenience may be improved.

<6. Exemplary variation>

Here, the disclosed embodiments are not limited to those described above, and a variety of variations are possible within a range that does not deviate from the scope and technical idea thereof.

In addition, the dispensing system 1 of the embodiments may be applied to preparation of medicines, for example, besides biochemical experiments in a biomedical field.

<7. Exemplary Hardware Configuration of Controller>

An exemplary hardware configuration will be described for the controllers 3, 4 achieving the processes of the first dispensing amount calculation part 44, the second dispensing amount calculation part 54, and the dispensing operation execution part 30 and so on implemented by a program executed by the CPU 901 described above, with reference to Fig. 12. The controllers 3, 4 includes the robot controller 3 and the host controller 4.

As shown in Fig. 12, the controllers 3, 4 has the circuitry including a CPU 901, a ROM 903, a RAM 905, a dedicated integrated circuit 907 constructed for specific use such as an ASIC or an FPGA, an input device 913, an output device 915, a storage device 917, a drive 919, a connection port 921, and a communication device 923. These constituent elements are mutually connected via a bus 909 and an I/O interface 911 such that signals can be transferred.

The program can be stored in a storage device such as the ROM 903, the RAM 905, and the storage device 917, for example.

The program can also temporarily or permanently be stored in a removable storage medium 925 such as magnetic disks including flexible disks, various optical disks including CDs, MO disks, and DVDs, and semiconductor memories. The removable storage medium 925 (a non-transitory recording medium) as described above can be provided as so-called packaged software. In this case, the program stored in the removable storage medium 925 may be read by the drive 919 and stored in the storage device 917 through the I/O interface 911 and the bus 909.

The program may be stored in, for example, a download site, another computer, or another storage device (not shown). In this case, the program is transferred through a network NW such as a LAN and the Internet and the communication device 923 receives this program. The program received by the communication device 923 may be stored in the storage device 917 through the I/O interface 911 and the bus 909.

The program may be stored in appropriate external connection device 927, for example. In this case, the program may be transferred through the appropriate connection port 921 and stored in the storage device 917 through the I/O interface 911 and the bus 909.

The CPU 901 executes various processes in accordance with the program stored in the storage device 917 to implement the processes of the first dispensing amount calculation part 44, the second dispensing amount calculation part 54, and the dispensing operation execution part 30 and so on. In this case, the CPU 901 may directly read and execute the program from the storage device 917 or may be execute the program once loaded in the RAM 905. In the case that the CPU 901 receives the program through, for example, the communication device 923, the drive 919, or the connection port 921, the CPU 901 may directly execute the received program without storing in the storage device 917.

The CPU 901 may execute various processes based on a signal or information input from the input device 913 such as a mouse, a keyboard, and a microphone (not shown) as needed.

The CPU 901 may output a result of execution of the process from the output device 915 such as a display device and a sound output device, for example, and the CPU 901 may transmit this process result through the communication device 923 or the connection port 921 as needed or may store the process result into the storage device 917 or the removable storage medium 925.

**Claims**

1. A dispensing system (1) comprising an automatic dispensing machine (2) configured to perform a dispensing work related to a stock solution (15) of a container (12), wherein

the dispensing system (1) comprises
a measured data acquisition part (41) configured to acquire measured data of concentration of the stock solution (15) including a matter (18), and
a target amount of matter acquisition part (53) configured to acquire a target amount of matter of matter (18) included in the stock solution (15); and
a second dispensing amount calculation part (54) configured to calculate a second dispensing amount of the stock solution (15) such that the matter (18) as much as the target amount of matter is extracted from the container (12), based on the measured data of concentration and the target amount of matter, and
a dispensing operation execution part (30) configured to control the automatic dispensing machine (2) to perform a first dispensing operation that dispenses the stock solution (15) as much as the second dispensing amount from the container (12) into other containers (20, 22) based on the measured data of concentration;
**characterised in that**:

the other containers (20, 22) include a single-amount container (20) into which a single-amount of the second dispensing amount of the stock solution (15) is dispensed and a N-fold amount container (22) into which an N-fold amount of the second dispensing amount of the stock solution (15) is dispensed,
wherein the automatic dispensing machine (2) comprises a pipette (8), and
wherein the dispensing system (1) further comprises a capacity determination part (55) configured to determine whether the second dispensing amount falls within a capacity range of the pipette (8),
wherein the dispensing operation execution part (30) controls the automatic dispensing machine (2) to perform the first dispensing operation that dispenses the single-amount of the second dispensing amount of the stock solution (15) from the container (12) into the single-amount container (20) when the second dispensing amount falls within the capacity range of the pipette (8) and dispenses the N-fold amount of the second dispensing amount of the stock solution (15) from the container (12) into the N-fold amount container (22) when the second dispensing amount falls outside the capacity range of the pipette (8), and controls the automatic dispensing machine (2) to perform a second dispensing operation that dispenses a content as much as 1/N of a total amount of the N-fold amount container (22) into the single-amount container (20), and
wherein the container (12) is each of a plurality of receiving parts (12) of a reservoir (10) including the plurality of receiving parts (12).

2. The dispensing system (1) according to claim 1, further comprising:

the target concentration acquisition part (43) configured to acquire a target concentration of the stock solution (15); and
a first dispensing amount calculation part (44) configured to calculate a first dispensing amount of diluent (16) into the container (12) such that the stock solution (15) reaches the target concentration, based on the measured data and the target concentration,
wherein the dispensing operation execution part (30) controls the automatic dispensing machine (2) to perform the dispensing operation that dispenses the diluent (16) as much as the first dispensing amount into the container (12).

3. The dispensing system (1) according to claim 1 or 2, further comprising:
an execution result storage part (48) configured to store an execution result of the dispensing operation in each of the plurality of receiving parts (12).

4. A dispensing method for controlling an automatic dispensing machine (2) to perform a dispensing work related to stock solution (15) of a container (12), comprising:

acquiring measured data of concentration of the stock solution (15) including a matter (18);
acquiring a target amount of matter of the matter (18) included in the stock solution (15);

calculating a second dispensing amount of the stock solution (15) such that the matter (18) as much as the target amount of matter is extracted from the container (12), based on the measured data of concentration and the target amount of matter, and

controlling the automatic dispensing machine (2) to perform a first dispensing operation that dispenses the stock solution (15) as much as the second dispensing amount from the container (12) into other containers (20, 22) based on the measured data of concentration,

**characterised in that**:

the other containers (20, 22) include a single-amount container (20) into which a single-amount of the second dispensing amount of the stock solution (15) is dispensed and a N-fold amount container (22) into which an N-fold amount of the second dispensing amount of the stock solution (15) is dispensed,

wherein the automatic dispensing machine (2) comprises a pipette (8), and

wherein the dispensing method further comprises determining whether the second dispensing amount falls within a capacity range of the pipette (8),

wherein the controlling the automatic dispensing machine (2) comprises controlling the automatic dispensing machine (2) to perform the first dispensing operation that dispenses the single-amount of the second dispensing amount of the stock solution (15) from the container (12) into the single-amount container (20) when the second dispensing amount falls within the capacity range of the pipette (8) and dispenses the N-fold amount of the second dispensing amount of the stock solution (15) from the container (12) into the N-fold amount container (22) when the second dispensing amount falls outside the capacity range of the pipette (8), and controlling the automatic dispensing machine (2) to perform a second dispensing operation that dispenses a content as much as 1/N of a total amount of the N-fold amount container (22) into the single-amount container (20), and

wherein the container (12) is each of a plurality of receiving parts (12) of a reservoir (10) including the plurality of receiving parts (12).

**Patentansprüche**

1. Ausgabesystem (1), welches eine Automatische-Ausgabe-Maschine (2) aufweist, welche konfiguriert ist, um eine Ausgabearbeit durchzuführen, welche sich auf eine Stammlösung (15) eines Behälters (12) bezieht, wobei das Ausgabesystem (1) aufweist:

ein Gemessene-Daten-Erlangung-Teil (41), welches konfiguriert ist, um gemessene Konzentrationsdaten der Stammlösung (15), welche einen Stoff (18) aufweist, zu erlangen, und ein Stoff-Zielmenge-Erlangung-Teil (53), welches konfiguriert ist, um eine Stoff-Zielmenge des Stoffs (18), welcher in der Stammlösung (15) enthalten ist, zu erlangen; und ein Zweite-Ausgabemenge-Berechnung-Teil (54), welches konfiguriert ist, um eine zweite Ausgabemenge der Stammlösung (15) zu berechnen derart, dass der Stoff (18) in Höhe der Stoff-Zielmenge aus dem Behälter (12) entnommen wird, basierend auf den gemessenen Konzentrationsdaten und der Stoff-Zielmenge, und ein Ausgabe-Vorgang-Ausführung-Teil (30), welches konfiguriert ist, um die Automatische-Ausgabe-Maschine (2) zu steuern, um einen ersten Ausgabevorgang durchzuführen, welcher die Stammlösung (15) in Höhe der zweiten Ausgabemenge von dem Behälter (12) aus in andere Behälter (20, 22) hinein ausgibt, basierend auf den gemessenen Konzentrationsdaten;

**dadurch gekennzeichnet, dass**:

die anderen Behälter (20, 22) einen Einfache-Menge-Behälter (20), in welchen hinein eine einfache Menge der zweiten Ausgabemenge der Stammlösung (15) ausgegeben wird, und einen N-fache-Menge-Behälter (22) aufweisen, in welchen hinein eine N-fache Menge der zweiten Ausgabemenge der Stammlösung (15) ausgegeben wird,

wobei die Automatische-Ausgabe-Maschine (2) eine Pipette (8) aufweist, und wobei das Ausgabesystem (1) ferner ein Kapazitätsermittlungsteil (55) aufweist, welches konfiguriert ist, um zu ermitteln, ob die zweite Ausgabemenge in einen Fassungsvermögen-Bereich der Pipette (8) hineinfällt,

wobei der Ausgabe-Vorgang-Ausführung-Teil (30) die Automatische-Ausgabe-Maschine (2) steuert, um den ersten Ausgabevorgang durchzuführen, welcher die einfache Menge der zweiten Ausgabemenge der Stammlösung (15) von dem Behälter (12) aus in den Einfache-Menge-Behälter (20) hinein ausgibt, wenn die zweite Ausgabemenge in den Fassungsvermögen-Bereich der Pipette (8) hineinfällt, und die N-fache Menge der zweiten Ausgabemenge der Stammlösung (15) von dem Behälter (12) aus in den N-Fache-Menge-Behälter (22) hinein ausgibt, wenn die zweite Ausgabemenge aus dem Fassungsvermögen-Bereich der Pipette (8) herausfällt, und die Automatische-Ausgabe-Maschine (2) steuert, um einen zweiten Ausgabevorgang durchzuführen, welcher einen Inhalt in Höhe von 1/N einer Gesamtmenge des N-fache-Menge-Behälters (22) in den Einfache-Menge-Behälter (20) hinein ausgibt, und wobei der Behälter (12) jedes von mehreren Aufnahmeteilen (12) eines Reservoirs (10) ist, welches die mehreren Aufnahmeteile (12) aufweist.

2. Ausgabesystem (1) gemäß Anspruch 1, ferner aufweisend:

den Zielkonzentration-Erlangung-Teil (43), welcher konfiguriert ist, um eine Zielkonzentration der Stammlösung (15) zu erlangen; und ein Erste-Ausgabemenge-Berechnung-Teil (44), welches konfiguriert ist, um eine erste Aus-gabemenge zu berechnen von Verdünnungs-mittel (16) in den Behälter (12) hinein derart, dass die Stammlösung (15) die Zielkonzentration erreicht, basierend auf den gemessenen Daten und der Zielkonzentration, wobei das Ausgabe-Vorgang-Ausführung-Teil (30) die Automatische-Ausgabe-Maschine (2) steuert, um den Ausgabevorgang durchzuführen, welcher das Verdünnungsmittel (16) in Höhe der ersten Ausgabemenge in den Behälter (12) hinein ausgibt.

3. Ausgabesystem (1) gemäß Anspruch 1 oder 2, ferner aufweisend:
ein Ausführung-Ergebnis-Speicher-Teil (48), welches konfiguriert ist, um ein Ausführungsergebnis des Ausgabevorgangs in jedem der mehreren Aufnahmeteile (12) zu speichern.

4. Ausgabeverfahren zum Steuern einer Automatische-Ausgabe-Maschine (2), um eine Ausgabearbeit durchzuführen, welche sich auf eine Stammlösung (15) eines Behälters (12) bezieht, aufwei-send:

Erlangen von gemessenen Konzentrationsda-ten der Stammlösung (15), welche einen Stoff (18) aufweist;
Erlangen einer Stoff-Zielmenge des Stoffes (18), welcher in der Stammlösung (15) enthalten ist;
Berechnen einer zweiten Ausgabemenge der Stammlösung (15) derart, dass der Stoff (18) in Höhe der Stoff-Zielmenge aus dem Behälter (12) entnommen wird, basierend auf den gemessenen Konzentrationsdaten und der Stoff-Zielmenge, und
Steuern der Automatische-Ausgabe-Maschine (2), um einen ersten Ausgabevorgang durchzuführen, welcher die Stammlösung (15) in Höhe der zweiten Ausgabemenge von dem Behälter (12) aus in andere Behälter (20, 22) hinein ausgibt, basierend auf den gemessenen Konzentrationsdaten;
**dadurch gekennzeichnet, dass**:

die anderen Behälter (20, 22) einen Einfache-Menge-Behälter (20), in welchen hinein eine einfache Menge der zweiten Aus-gabemenge der Stammlösung (15) ausge-geben wird, und einen N-fache-Menge-Behälter (22) aufweisen, in welchen hinein eine N-fache Menge der zweiten Ausgabemenge der Stammlösung (15) ausgegeben wird,
wobei die Automatische-Ausgabe-Maschine (2) eine Pipette (8) aufweist, und wobei das Ausgabeverfahren ferner ein Ermitteln aufweist, ob die zweite Ausgabe-menge in einen Fassungsvermögenbe-reich der Pipette (8) hineinfällt,
wobei das Steuern der Automatische-Ausgabe-Maschine (2) aufweist: Steuern der Automatische-Ausgabe-Maschine (2), um den ersten Ausgabevorgang durchzuführen, welcher die einfache Menge der zweiten Ausgabemenge der Stammlösung (15) von dem Behälter (12) aus in den Einfache-Menge-Behälter (20) hinein ausgibt, wenn die zweite Ausgabemenge in den Fassungsvermögen-Bereich der Pipette (8) hineinfällt, und die N-fache Menge der zweiten Ausgabemenge der Stammlösung (15) von dem Behälter (12) aus in den N-fache-Menge-Behälter (22) hinein ausgibt, wenn die zweite Ausgabemenge aus dem Fassungsvermögen-Bereich der Pipette (8) herausfällt, und Steuern der Automatische-Ausgabe-Maschine (2), um einen zweiten Ausgabevorgang durchzuführen, welcher einen Inhalt in Höhe von 1/N einer Gesamtmenge des N-fache-Menge-Behälters (22) in den Einfache-Menge-Behälter (20) hinein ausgibt, und
wobei der Behälter (12) jedes von mehreren Aufnahmeteilen (12) eines Reservoirs (10) ist, welches die mehreren Aufnahmeteile (12) aufweist.

## Revendications

1. Système de distribution (1) comprenant une machine de distribution automatique (2) configurée pour effectuer un travail de distribution lié à une solution mère (15) d'un récipient (12), dans lequel :

    le système de distribution (1) comprend
    une partie d'acquisition de données mesurées (41) configurée pour acquérir des données mesurées de concentration de la solution mère (15) comprenant une matière (18), et
    une partie d'acquisition de quantité cible de matière (53) configurée pour acquérir une quantité cible de matière de la matière (18) incluse dans la solution mère (15) ; et
    une partie de calcul de deuxième quantité de distribution (54) configurée pour calculer une deuxième quantité de distribution de la solution mère (15) de telle sorte que la matière (18) soit extraite du récipient (12) en quantité égale à la quantité cible de matière, sur la base des données mesurées de concentration et de la quantité cible de matière, et
    une partie d'exécution d'opération de distribution (30) configurée pour commander la machine de distribution automatique (2) afin d'effectuer une première opération de distribution qui distribue la solution mère (15) en quantité égale à la deuxième quantité de distribution depuis le récipient (12) dans d'autres récipients (20, 22) sur la base des données mesurées de concentration ;
    **caractérisé en ce que** :

    les autres récipients (20, 22) comprennent un récipient de quantité unique (20) dans lequel une quantité unique de la deuxième quantité de distribution de la solution mère (15) est distribuée et un récipient de quantité de N fois (22) dans lequel une quantité de N fois de la deuxième quantité de distribution de la solution mère (15) est distribuée,
    dans lequel la machine de distribution automatique (2) comprend une pipette (8), et
    dans lequel le système de distribution (1) comprend en outre une partie de détermination de capacité (55) configurée pour déterminer si la deuxième quantité de distribution se situe dans une plage de capacité de la pipette (8),
    dans lequel la partie d'exécution d'opération de distribution (30) commande la machine de distribution automatique (2) pour effectuer la première opération de distribution qui distribue la quantité unique de la deuxième quantité de distribution de la solution mère (15) du récipient (12) dans le récipient de quantité unique (20) lorsque la deuxième quantité de distribution se situe dans la plage de capacité de la pipette (8) et distribue la quantité de N fois de la deuxième quantité de distribution de la solution mère (15) du récipient (12) dans le récipient de quantité de N fois (22) lorsque la deuxième quantité de distribution se situe en dehors de la plage de capacité de la pipette (8), et commande la machine de distribution automatique (2) pour effectuer une deuxième opération de distribution qui distribue un contenu en quantité égale à 1/N d'une quantité totale du récipient de quantité de N fois (22) dans le récipient de quantité unique (20), et
    dans lequel le récipient (12) est chacune d'une pluralité de parties de réception (12) d'un réservoir (10) comprenant la pluralité de parties de réception (12).

2. Système de distribution (1) selon la revendication 1, comprenant en outre :

    la partie d'acquisition de concentration cible (43) configurée pour acquérir une concentration cible de la solution mère (15) ; et
    une partie de calcul de première quantité de distribution (44) configurée pour calculer une première quantité de distribution de diluant (16) dans le récipient (12) de telle sorte que la solution mère (15) atteigne la concentration cible, sur la base des données mesurées et de la concentration cible,
    dans lequel la partie d'exécution d'opération de distribution (30) commande la machine de distribution automatique (2) pour effectuer l'opération de distribution qui distribue le diluant (16) en quantité égale à la première quantité de distribution dans le récipient (12).

3. Système de distribution (1) selon la revendication 1 ou 2, comprenant en outre :
    une partie de stockage de résultat d'exécution (48) configurée pour stocker un résultat d'exécution de l'opération de distribution dans chacune de la pluralité de parties de réception (12).

4. Procédé de distribution pour commander une machine de distribution automatique (2) afin d'effectuer un travail de distribution lié à une solution mère (15) d'un récipient (12), comprenant :

    l'acquisition de données mesurées de concentration de la solution mère (15) comprenant une matière (18) ;
    l'acquisition d'une quantité cible de matière de la matière (18) incluse dans la solution mère (15) ;
le calcul d'une deuxième quantité de distribution de la solution mère (15) de telle sorte que la matière (18) soit extraite du récipient (12) en quantité égale à la quantité cible de matière, sur la base des données mesurées de concentration et de la quantité cible de matière, et
la commande de la machine de distribution automatique (2) pour effectuer une première opération de distribution qui distribue la solution mère (15) en quantité égale à la deuxième quantité de distribution du récipient (12) dans d'autres récipients (20, 22) sur la base des données mesurées de concentration,

**caractérisé en ce que** :

les autres récipients (20, 22) comprennent un récipient de quantité unique (20) dans lequel une quantité unique de la deuxième quantité de distribution de la solution mère (15) est distribuée et un récipient de quantité de N fois (22) dans lequel une quantité de N fois de la deuxième quantité de distribution de la solution mère (15) est distribuée,

dans lequel la machine de distribution automatique (2) comprend une pipette (8), et

dans lequel le procédé de distribution comprend en outre la détermination du fait que la deuxième quantité de distribution se situe dans une plage de capacité de la pipette (8),

dans lequel la commande de la machine de distribution automatique (2) comprend la commande de la machine de distribution automatique (2) pour effectuer la première opération de distribution qui distribue la quantité unique de la deuxième quantité de distribution de la solution mère (15) du récipient (12) dans le récipient de quantité unique (20) lorsque la deuxième quantité de distribution se situe dans la plage de capacité de la pipette (8) et distribue la quantité de N fois de la deuxième quantité de distribution de la solution mère (15) du récipient (12) dans le récipient de quantité de N fois (22) lorsque la deuxième quantité de distribution se situe en dehors de la plage de capacité de la pipette (8), et la commande de la machine de distribution automatique (2) pour effectuer une deuxième opération de distribution qui distribue un contenu en quantité égale à 1/N d'une quantité totale du récipient de quantité de N fois (22) dans le récipient de quantité unique (20), et

dans lequel le récipient (12) est chacune d'une pluralité de parties de réception (12) d'un réservoir (10) comprenant la pluralité de parties de réception (12).

[FIG. 1]

5 MEASURING INSTRUMENT

MEASURED DATA

4 HOST CONTROLLER

DISPENSING COMMAND

3 ROBOT CONTROLLER

30 DISPENSING OPERATION EXECUTION PART

31 ROBOT CONTROL PART

32 PIPETTE CONTROL PART

1

2

2a

8

7

Z

X Y

10

9

6

EP 3 462 177 B1

EP 3 462 177 B1

[FIG. 2]

[FIG. 3]

18

[FIG. 4]

HOST CONTROLLER (4)

FROM MEASURING INSTRUMENT 5 →

MEASURED DATA ACQUISITION PART (41)

TARGET CONCENTRATION ACQUISITION PART (43)

SETTING FILE ACQUISITION PART (42)

FIRST DISPENSING AMOUNT CALCULATION PART (44)

COMMAND STORAGE PART (45)

JOB GENERATION PART (46)

JOB EXECUTION PART (47)

EXECUTION RESULT STORAGE PART (48)

DISPENSING COMMAND

ROBOT CONTROLLER (3)

DISPENSING OPERATION EXECUTION PART (30)

PIPETTE CONTROL PART (32)

ROBOT CONTROL PART (31)

→ ELECTRIC PIPETTE (8)

→ ROBOT (2)

[FIG. 5]

14A

TARGET
CONCENTRATION: b

| ADDRESS | STOCK SOLUTION AMOUNT | CONCENT-RATION | DILUENT DISPENSING AMOUNT |
|---|---|---|---|
| A1 | c1 | a1 | d1 |
| B1 | c2 | a2 | d2 |
| C1 | c3 | a3 | d3 |
| D1 | c4 | a4 | d4 |
| E1 | c5 | a5 | Skip |
| ⋮ | ⋮ | ⋮ | ⋮ |

17

16

A1  B1  C1  D1  E1

12

15

A1  B1  C1  D1  E1

12

[FIG. 6]

[FIG. 7A]

14A

| ADDRESS | STOCK SOLUTION AMOUNT | CONCENT-RATION | DILUENT DISPENSING AMOUNT |
|---|---|---|---|
| A1 | c1 | | |
| B1 | c2 | | |
| C1 | c3 | | |
| D1 | c4 | | |
| E1 | c5 | | |
| ⋮ | ⋮ | | |

[FIG. 7B]

14A    TARGET CONCENTRATION: b

| ADDRESS | STOCK SOLUTION AMOUNT | CONCENT-RATION | DILUENT DISPENSING AMOUNT |
|---|---|---|---|
| A1 | c1 | a1 | d1 |
| B1 | c2 | a2 | d2 |
| C1 | c3 | a3 | d3 |
| D1 | c4 | a4 | d4 |
| E1 | c5 | a5 | Skip |
| ⋮ | ⋮ | ⋮ | ⋮ |

[FIG. 7C]

14A

TARGET CONCENTRATION: b

| ADDRESS | STOCK SOLUTION AMOUNT | CONCENT-RATION | DILUENT DISPENSING AMOUNT | EXECUTION RESULT |
|---------|------------------------|----------------|----------------------------|-------------------|
| A1 | c1 | a1 | d1 | DONE |
| B1 | c2 | a2 | d2 | DONE |
| C1 | c3 | a3 | d3 | DONE |
| D1 | c4 | a4 | d4 | DONE |
| E1 | c5 | a5 | Skip | UNDONE |
| ⋮ | ⋮ | ⋮ | ⋮ | ⋮ |

[FIG. 8]

[FIG. 9]

14B — TARGET AMOUNT OF MATTER: f

| ADDRESS | STOCK SOLUTION AMOUNT | CONCENT- RATION | DILUENT DISPENSING AMOUNT |
|---|---|---|---|
| A1 | g1 | a1 | e1 |
| B1 | g2 | a2 | e2 |
| C1 | g3 | a3 | e3 |
| D1 | g4 | a4 | Skip |
| E1 | g5 | a5 | e5 |
| ... | ... | ... | ... |

[FIG. 10]

```
                    ┌─────────────┐
                    │    START    │
                    └─────────────┘
                           │
                           ▼
              ┌──────────────────────────┐
              │    READ SETTING FILE     │ ─── S110
              └──────────────────────────┘
                           │
                           ▼
              ┌──────────────────────────┐
              │   ACQUIRE TARGET AMOUNT  │ ─── S120
              │        OF MATTER         │
              └──────────────────────────┘
                           │
                           ▼
              ┌──────────────────────────┐
              │   ACQUIRE MEASURED DATA  │ ─── S130
              │      (CONCENTRATION)     │
              └──────────────────────────┘
                           │
                           ▼
              ┌──────────────────────────┐
              │   CALCULATE DISPENSING   │ ─── S140
              │  AMOUNT OF STOCK SOLUTION│
              └──────────────────────────┘
```

S150

WITHIN PIPETTE CAPACITY?    NO

YES

S160

DISPENSE STOCK SOLUTION INTO SINGLE-AMOUNT CONTAINER BASED ON CALCULATED DISPENSING AMOUNT

S170

DISPENSE STOCK SOLUTION INTO N-FOLD-AMOUNT CONTAINER BASED ON CALCULATED DISPENSING AMOUNT

S180

COMPLETION OF ALL WELLS?    NO

YES

DISPENSE STOCK SOLUTION AS MUCH AS 1/N OF TOTAL AMOUNT FROM N-FOLD AMOUNT CONTAINER INTO SINGLE-AMOUNT CONTAINER ─── S190

STORE EXECUTION RESULT ─── S200

END

[FIG. 11A]

14B

| ADDRESS | STOCK SOLUTION AMOUNT | CONCENT- RATION | STOCK SOLUTION DISPENSING AMOUNT |
|---|---|---|---|
| A1 | g1 | | |
| B1 | g2 | | |
| C1 | g3 | | |
| D1 | g4 | | |
| E1 | g5 | | |
| ⋮ | ⋮ | | |

[FIG. 11B]

14B    TARGET AMOUNT OF MATTER: f

| ADDRESS | STOCK SOLUTION AMOUNT | CONCENT- RATION | STOCK SOLUTION DISPENSING AMOUNT |
|---|---|---|---|
| A1 | g1 | a1 | e1 |
| B1 | g2 | a2 | e2 |
| C1 | g3 | a3 | e3 |
| D1 | g4 | a4 | Skip |
| E1 | g5 | a5 | e5 |
| ⋮ | ⋮ | ⋮ | ⋮ |

[FIG. 11C]

14B

TARGET AMOUNT
OF MATTER: f

| ADDRESS | STOCK SOLUTION AMOUNT | CONCENT-RATION | STOCK SOLUTION DISPENSING AMOUNT | EXECUTION RESULT |
|---------|------------------------|----------------|-----------------------------------|------------------|
| A1 | g1 | a1 | e1 | DONE |
| B1 | g2 | a2 | e2 | DONE |
| C1 | g3 | a3 | e3 | DONE |
| D1 | g4 | a4 | Skip | UNDONE |
| E1 | g5 | a5 | e5 | DONE |
| ⋮ | ⋮ | ⋮ | ⋮ | ⋮ |

[FIG. 12]

3,4

CONTROLLER

901
CPU

903
ROM

905
RAM

907
DEDICATED
INTEGRATED CIRCUIT

909
BUS

911
INPUT/OUTPUT INTERFACE

913
INPUT
DEVICE

915
OUTPUT
DEVICE

917
STORAGE
DEVICE

919
DRIVE

921
CONNECTION
PORT

923
COMMUNICATION
DEVICE

STORAGE
MEDIUM
925

EXTERNAL
CONNECTION
DEVICE
927

NW

EP 3 462 177 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009130318 A

- US 2008072664 A1